# EUROPEAN PATENT APPLICATION

(11) **EP 2 544 113 A1**
(43) Date of publication of application: **09.01.2013**
(21) Application number: 11174187.2
(22) Date of filing: 15.07.2011
(51) Int. Cl.: G06F 19/26

(54) **Genomic/proteomic sequence representation, visualization, comparison and reporting using a bioinformatics character set and a mapped bioinformatics font**

(30) Priority: 05.07.2011 US 504411 P
(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

Genomic or proteomic data are encoded as a genomic or proteomic character string comprising characters of a bioinformatics character set **(20)**. Each base or peptide of the genomic or proteomic data is represented by a single character of the bioinformatics character set, and each character of the bioinformatics character set encodes (I) a base or peptide and (II) at least one annotated datum value associated with the base or peptide. The genomic or proteomic data are displayed by displaying the genomic or proteomic character string using a bioinformatics font **(40)** mapped to the bioinformatics character set. At least one string function may be performed on the genomic or proteomic character string to generate an updated genomic or proteomic character string in which at least one base or peptide is represented by a single character encoding at least one additional or modified annotated datum generated by the performed string manipulation.

## Description

The following relates to bioinformatics, genomic processing arts, proteomic processing arts, and related arts.

Genomic or proteomic data comprises an ordered sequence of bases or peptides. In the case of deoxyribonucleic acid (DNA) the bases are adenine, cytosine, guanine, and thymine, which are commonly represented by the letters "A", "C", "G", and "T", respectively. In the case of ribonucleic acid (RNA) the bases are adenine, cytosine, guanine, and uracil, which are commonly represented by the letters "A", "C", "G", and "U", respectively. DNA and RNA differ by the substitution of uracil (U) for thymine (T).

Proteins and other proteomic molecules comprise amino acids connected by peptide bonds. The corresponding proteomic data are suitably represented by a peptide (or amino acid) sequence. (The terms "peptide" and "amino acid" are used interchangeably herein to refer to elements of a proteomic sequence). The amino acids are typically represented by a one-letter or three-letter code. For example: alanine is represented by the letter "A" or the three letter code "Ala"); arginine is represented by the letter "R" or the three letter code "Arg"; and so forth. In a peptide sequence, the individual peptides are typically denoted by their amino acid constituents, as the peptide bond does not change. Thus, for example, "A" or "Ala" is used in a protein sequence to denote the peptide comprising alanine.

Genomic or proteomic data contains substantial useful information, which is typically extracted by pattern matching. For example, the genomic or proteomic data may be searched for a disease marker that has been correlated with a certain disease in past clinical studies, or the data may be used for therapy planning for certain diseases such as cancers, metabolic disorders, or so forth. Genetic markers indicative of ancestral lineages can be used to assess the ancestry of a subject. In law enforcement or certain other fields, correlation of phenotype with genotype can provide useful information. For example, a DNA sample obtained from the scene of a crime may indicate that the criminal has a certain physical characteristic, thus eliminating any suspect who does not have that characteristic.

Sequence processing typically entails the following operations: acquisition of sequence fragments; alignment of the sequence fragments respective to a reference sequence (for example, some suitable reference sequences include: RefSeq, hg18, hg19, Pan-Genome, and so forth); and analysis of the aligned sequence to identify subject-specific variations. It may also include de novo alignment where there is no reference to map, leading to generation of contigs of multiple lengths which can then be annotated and comparative analysis carried out.

The genomic or proteomic data are typically acquired as sequence fragments that are stored in a standardized format such as FastA or FastQ. One suitable line of sequencer systems that output FastA or FastQ data are the Illumina sequencers (available from Illumina Inc., San Diego, CA, USA). The FastA format uses a single letter to represent each base or peptide (e.g., the base representations "A", "C", "G", and "T" for DNA, or "A", "C", "G", and "U" for RNA). Additional single-letter codes may be provided to represent ambiguous bases - for example, the letter "R" may represent an ambiguous base which is either adenine or guanine. The FastQ format is an extension of FastA that includes an additional line of characters representing the quality values for the bases of the sequence. A FastQ file is about twice as large as the corresponding FastA file, due to the use of two characters for each base (one character to represent the base value, and a second character to represent the quality value).

The sequence fragments are aligned with respect to a reference sequence to generate aligned genomic or proteomic data that are typically stored in a Sequence Alignment/Map (SAM) text file or an equivalent binary BAM file. The SAM format conventionally employs a "1-based" coordinate system in which the coordinate of the first base of a sequence is one, whereas the BAM format conventionally employs a "0-based" coordinate system in which the coordinate of the first base of a sequence is zero. The sequence fragments forming the aligned sequence suitably remain in the FastA format (converted to a binary format in the case of BAM). Sequence alignment enables identification of functional regions such as genes, introns (extraneous sub-sequences within a gene that are not translated into a protein), exons (the portions of a gene that are translated into a protein), promoters (sub-sequences that facilitate gene transcription), sequences coding for transcription factor (TF) binding sites (TF proteins bind with TF binding sites in the DNA sequence to control transcription), sequences transcribing to non-coding RNA (nc-RNA), and so forth.

The aligned genomic or proteomic sequence is then analyzed to identify variations, such as single nucleotide polymorphism (SNP), copy number variation (CNV), sub-sequence insertion or deletion (indel) features, various intrachromosomal and/or interchromosomal rearrangements, and so forth. These variations may serve as disease markers, ancestral lineage markers, or so forth.

Sequence processing is computationally intensive and generates large quantities of product data. For illustration, a typical genome sequencing study may generate about 40 GB of FastA data, and/or about 80 GB FastQ data. Alignment of these sequence fragments generates one or more SAM files of around 200 GB (reducible to about 100 GB in binary BAM format). The aligned data are processed by pattern matching algorithms to identify variations of interest, and the results of these studies are typically stored as various text files, tables, spreadsheets, or other data compilations.

These existing approaches have certain disadvantages. A physician or other reviewer may need to access and review numerous files in order to obtain desired information. The variational analyses themselves may also be complicated by the diversity of data storage. For example, consider a variation that is probative when it occurs in an exon, but not elsewhere. In the variational analysis, pattern matching is first performed to identify a candidate sequence matching the variation signature. Thereafter, the source SAM file is accessed to determine whether the candidate sequence match occurs in an exon. This variational analysis involves two steps: (1) matching the base sequence; and (2) matching the base sequence match to an exon. (Alternatively, the exon data could first be identified using the SAM content, followed by base sequence matching applied only to the exon data. Again, however, this is a two-step process).

The presentation of sequencing results is also complicated by the diversity of data storage. Typically, results of each variational analysis are stored in its own text file, table, spreadsheet, or other data compilation. The physician or other reviewer is thus required to review the different data compilations to assess the results of the study. This process may miss synergistic or discordant combinations of information. The textual format of the tables and/or spreadsheets may also be difficult to comprehend. One solution is to additionally present selected results in the form of a graph, color-coded chart, or the like. However, the generation of such auxiliary data representations further increases computational complexity. Moreover, graphical result summaries may disconnect these results from the underlying genomic or proteomic sequence data.

The following provides new and improved apparatuses and methods as disclosed herein.

In accordance with one disclosed aspect, a method comprises: encoding genomic or proteomic data as a genomic or proteomic character string comprising characters of a bioinformatics character set wherein: (i) each base or peptide of the genomic or proteomic data is represented by a single character of the bioinformatics character set and (ii) each character of the bioinformatics character set encodes (I) a base or peptide and (II) at least one annotated datum value associated with the base or peptide; and displaying the genomic or proteomic data by displaying the genomic or proteomic character string using a bioinformatics font mapped to the bioinformatics character set. The encoding and displaying are suitably performed by a digital processing device. The method may further include performing at least one string function on the genomic or proteomic character string to generate an updated genomic or proteomic character string in which at least one base or peptide is represented by a single character encoding at least one additional or modified annotated datum generated by the performed string manipulation.

In accordance with another disclosed aspect, a digital processing device is configured to perform a method as set forth in the immediately preceding paragraph. In accordance with another disclosed aspect, a non-transitory storage medium is readable by a digital processing device and stores instructions executable by the digital processing device to perform a method as set forth in the immediately preceding paragraph.

In accordance with another disclosed aspect, a non-transitory storage medium is readable by a digital processor and stores software for processing genomic or proteomic data represented as genomic or proteomic character strings comprising characters of a bioinformatics character set wherein each base or peptide of the genomic or proteomic data is represented by a single character of the bioinformatics character set and the characters of the bioinformatics character set encode bases or peptides and additional data associated with the bases or peptides. In some embodiments the software processes the genomic or proteomic data using string processing operations. In some embodiments the software processes the genomic or proteomic data using bitwise masking operations to zero selected binary bits of characters representing bases or peptides. In some embodiments the storage medium further stores a bioinformatics font mapped to the bioinformatics character set, and the software performs display operations in which genomic or proteomic data are displayed using the bioinformatics font.

One advantage resides in more compact and centralized storage of genomic or proteomic data.

Another advantage resides in storing base quality values, functional region information, variational information, or other annotated data with the base or peptide sequence in a compact single character representation.

Another advantage resides in providing an intuitive display of a base or peptide sequence including diacritical marks or other font features denoting or delineating annotated information such as base quality values, functional regions, variations, or so forth.

Another advantage resides in comparing genomic sequences using conventional character string operations. The comparing of the strings may be readily configured to detect only particular changes in annotation (for example, Methylation)

Other advantages include facilitating comparison of different annotations of the reference sequence annotated by different research groups and ease of visualization of epigenetic changes in normal versus cancer genomes.

Further advantages will be apparent to those of ordinary skill in the art upon reading and understanding the following detailed description.
FIGURE 1 diagrammatically shows a sequence alignment/analysis module employing a bioinformatics character set and mapped bioinformatics font.
FIGURE 2 tabulates a suitable diacritical marking system for depicting base quality scores along with a base sequence.
FIGURE 3 shows a depiction of a portion of a base sequence including diacritical marks delineated coding sequence (CDS) start and end codons.
FIGURE 4 tabulates some suitable diacritical marks for depicting regional and variation information annotated to bases.
FIGURE 5 shows a depiction of a portion of a base sequence including selected diacritical marks delineating an intron disposed between two exons.
FIGURE 6 diagrammatically shows an approach for constructing a font character of the bioinformatics font by combining a bitmap for the base with bitmaps for diacritical marks using a bitwise OR operation.
FIGURE 7 shows a depiction of a portion of a base sequence including diacritical marks delineated coding sequence (CDS) start and end codons and diacritical marks delineating an intron, using the diacritical marks of the bioinformatics font embodiment of FIGURE 6.
FIGURE 8 diagrammatically shows a portion of a base sequence displayed along with check boxes via which a reviewer can select which annotation types are to be depicted in the displayed base sequence.
FIGURE 9 diagrammatically shows an approach for constructing a font character of a bioinformatics font for representing amino acids along with their biophysical properties by combining a bitmap for the amino acid with bitmaps for diacritical marks using a bitwise OR operation.

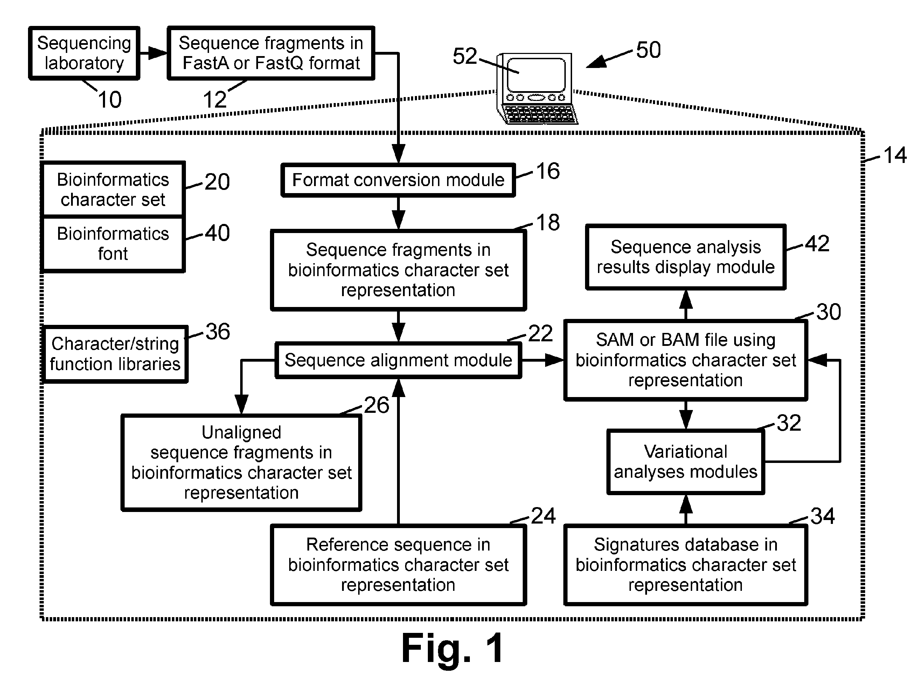

With reference to FIGURE 1, a genomic or proteomic sequencing analysis system includes a sequencing laboratory **10** that prepares and sequences a sample of DNA, RNA, protein, or so forth to produce base sequence fragments (for a genomic sample) or peptide sequence fragments (for a proteomic sample) that are stored as sequence fragments in a FastA or FastQ format **12**. By way of illustrative example, a sample of DNA material may be prepared and sequenced by operations such as DNA purification and cloning, amplification by techniques such as polymerase chain reaction (PCR) employing fluorescently labeled chain terminators such as dideoxynucleotide triphosphate (ddNTP) terminators, and sequencing by capillary electrophoresis or another sequencing technique. In the laboratory **10** these various operations may be performed as manual, semi-automated, or fully automated processing operations. For example, certain sample preparation operations may be performed manually or in a semi-automated fashion, followed by loading and sequencing of the prepared sample using an automated sequencing apparatus. Additionally or alternatively, the laboratory **10** may perform proteomic sequencing using Edman degradation and mass spectrometry or another suitable technique.

The output of the illustrative sequencing laboratory **10** comprises sequence fragments in FastA or FastQ format **12**. These are conventional formats. In FastA, a sequence of bases or peptides is represented by a sequence of characters wherein each character represents one base or peptide. For example, the genomic sequence "adenine-guanine-cytosine" is suitably represented in FastA as "AGC". In the FastQ format, a second string is added that parallels the string representing the bases or peptides. The second string represents the quality value for each base or peptide using a single-character code. Thus, in FastQ each base or peptide is represented by two characters: a first character in the first (e.g., base sequence) string giving the base or peptide identity, and a second character in the second (e.g., quality values) string giving the quality value for that base or peptide output by the automated sequencer (or other suitable quality assessment).

With continuing reference to FIGURE 1, a sequence alignment/analysis module **14** receives the sequence fragments **12**. A format conversion module **16** converts the FastA or FastQ sequences into a single-string representation **18** employing a bioinformatics character set **20.** As disclosed herein, the bioinformatics character set **20** is designed to represent genomic or proteomic sequences in a compact single-string format in which each base or peptide is represented by a single character of the bioinformatics character set **20**. That single character encodes both the base or peptide and annotated data associated with the base or peptide.

It is recognized herein that existing formats such as FastA are not compact. For example, consider encoding to genomic data. There are four encoding values needed to encode the four bases (assuming either thymine or uracil, but not both). These four possibilities can be encoded with as few as two bits. If ambiguity is also encoded, then this can require as many as fifteen possible values: the four "known" bases, six additional ambiguous "two-base" combinations (e.g., a site known to be either adenine or cytosine), four possible ambiguous "three-base" combinations (e.g., a site known to be adenine, cytosine, or guanine), and one completely ambiguous combination (that is, a site that could by any of adenine, cytosine, guanine, or thymine). These fifteen possible values can be encoded with only four bits. If less detailed ambiguity is to be encoded, fewer possible values are needed. For example, if only the four bases and a single ambiguous "N" code is used, then there are only five possible values which can be encoded with three bits. However, FastA employs a full byte (eight bits) to represent the information.

The one-byte encoding scheme of FastA does have substantial advantages. The single-byte approach comports with conventional digital processor architecture in which data are organized into byte units of eight bits each. Moreover, the "A", "C", "G", "T" characters (and optional ambiguous characters such as "N") conform with the American Standard Code for Information Interchange (ASCII) character set, and accordingly existing string functions can be employed to manipulate FastA data. But, it is recognized herein that FastA "wastes" a substantial amount of the encoding power of each byte. The eight bits of one byte can store 256 possible values (ranging from 0-255); whereas, FastA uses only fifteen (or fewer) possible combinations.

FastQ is an extension of FastA. The base-encoding (or peptide-encoding) string of a FastQ-formatted file is identical with that of FastA, and so the foregoing remarks apply to FastQ as well. Additionally, FastQ includes a second string containing the base quality values, with one byte used for each quality value. If phred quality scores are encoded, these scores range from 0-93. The 94 possible values can be encoded with as few as 7 bits, but FastQ uses a full 8-bit byte to encode the quality values.

In contrast, embodiments of the bioinformatics character set **20** disclosed herein provide more compact storage that has additional advantages. The bioinformatics character set **20** employs a single character to represent each base or peptide of a sequence. To retain the advantage of FastA and FastQ in terms of comporting with existing digital processing architectures, each character is typically a single byte or two bytes. Designing the bioinformatics character set **20** to employ a single byte for each character means that the character set comports well with standard ASCII, which also employs a single byte per character. On the other hand, embodiments in which the bioinformatics character set **20** employs two bytes for each character comport well with standard Unicode, which employs two bytes per character.

The bioinformatics character set **20** is not, however, ASCII or Unicode. Rather, the bioinformatics character set **20** is designed to employ one sub-set of bits of the character to represent the base or peptide, and to employ another sub-set of bits of the character to represent at least one annotated datum value (and typically several different annotated data values) associated with the base or peptide.

By way of illustrative example, Table 1 sets forth an embodiment of the bioinformatics character set **20** which is suitable for representing genomic data (and more specifically DNA sequences) using a single character per base with each character being a two-byte character having sixteen bits. The bits of a single character are suitably written as b₁₅ b₁₄ b₁₃ b₁₂ b₁₁ b₁₀ b₉ b₈ b₇ b₆ b₅ b₄ b₃ b₂ b₁ b₀ where b₁₅ is the most significant bit and b₀ is the least significant bit. The most significant byte comprises the bits b₁₅ b₁₄ b₁₃ b₁₂ b₁₁ b₁₀ b₉ b₈ while the least significant byte comprises the bits b₇ b₆ b₅ b₄ b₃ b₂ b₁ b₀. In the bioinformatics character set **20** of Table 1 bits b₂ b₁ b₀ are used to represent the base. There are five allowable values: four values for representing the four bases of DNA, and a fifth "ambiguous" value representing an unknown base. The remaining 13 bits b₁₅ b₁₄ b₁₃ b₁₂ b₁₁ b₁₀ b₉ b₈ b₇ b₆ b₅ b₄ b₃ are used to represent various annotated data values associated with the base. (See Table 1 for illustrative examples).

The format conversion performed by the format conversion module **16** in the case of the illustrative embodiment of the bioinformatics character set **20** of Table 1 suitably operates as follows: FastA code "A" converts to character 0000000000000000_{bin} (0000ₕₑₓ); FastA code "C" converts to character 0000000000000001_{bin} (0001ₕₑₓ); FastA code "G" converts to character 0000000000000010_{bin} (0002ₕₑₓ); FastA code "T" converts to character 0000000000000011_{bin} (0003ₕₑₓ); and all other FastA codes corresponding to ambiguous bases converts to character 0000000000000100_{bin} (0004ₕₑₓ). FastQ is converted in the same way in this embodiment, except that bits b₆ b₅ b₄ b₃ are also filled in with the encoding of the quality value in accordance with the encoding scheme shown in Table 1. Note that this encoding scheme enables the phred score to be represented with only four bits, albeit with some loss of resolution (e.g., b₆ b₅ b₄ b₃ = 0100 specifies the phred score only as being in the range 31-40). Typically, one only wants to know if the quality score is "high" or "low", and so this loss of quality value resolution is generally not problematic.

The conversion performed by the format conversion module **16** in the case of the illustrative embodiment of the bioinformatics character set **20** of Table 1 sets the remaining annotation bits b₁₅ b₁₄ b₁₃ b₁₂ b₁₁ b₁₀ b₉ b₈ b₇ to a default value of zero. This reflects the reality that alignment and variational analysis has not yet been performed and thus there are no bases identified as belonging to exons, introns, or so forth.

In the illustrative embodiment of FIGURE 1, the sequencing laboratory **10** generates data **12** in FastA or FastQ format which is then converted by the format conversion module **16** to the data representation **18** in using the bioinformatics character set **20**. This approach advantageously enables the sequence alignment/analysis module **14** to process sequencing fragments data generated in a conventional FastA or FastQ format. Alternatively, however, the sequencing laboratory **10** can directly output the sequence fragments in the representation of the bioinformatics character set **20**.

With continuing reference to FIGURE 1, the sequence fragments **18** in the representation of the bioinformatics character set **20** are processed by a sequence alignment module **22**. The sequence alignment module operates to align sequence fragments, typically with reference to a reference sequence **24**, in order to "splice together" the sequence fragments to form a (more) complete and long(er) aligned sequence. The alignment processing is analogous to that conventionally applied for FastA sequence fragments, i.e. matching ends of sequence fragments in order to align and splice them together. However, with the data representation **18** the annotation bits (e.g., quality value annotation b₆ b₅ b₄ b₃ in the example of Table 1) could result in mismatches. In other words, two identical bases having different quality values might not match because of the different quality values.

**TABLE 1**

| **Bioinformatics character** | **Quantity stored** | **Allowable values** |
|---|---|---|
| **b₁₅ b₁₄ b₁₃ ··· b₃ b₂ b₁ b₀** | | |
| b₂ b₁ b₀ | Base | 000=adenine; 001=cytosine; |
| | | 010=guanine; 011=thymine; |
| | | 100=Unknown |
| b₆ b₅ b₄ b₃ | Base quality | 0000 = phred score 0 |
| | | 0001 = phred score 1-10 |
| | | 0010 = phred score 11-20 |
| | | 0011 = phred score 21-30 |
| | | 0100 = phred score 31-40 |
| | | 0101 = phred score 41-50 |
| | | 0110 = phred score 51-60 |
| | | 0111 = phred score 61-70 |
| | | 1000 = phred score 71-80 |
| | | 1001 = phred score 81-90 |
| | | 1010 = phred score > 90 |
| b₇ | Coding sequence (CDS) start | 1=yes ; 0=no |
| b₈ | Coding sequence (CDS) end | 1=yes ; 0=no |
| b₉ | Exon | 1=part of exon ; |
| | | 0=not part of exon |
| b₁₀ | Intron | 1=part of intron ; |
| | | 0=not part of intron |
| b₁₁ | Promoter | 1=part of promoter; |
| | | 0=not part of promoter |
| bₗ₂ | Transcription factor (TF) | 1=part of TF binding site; |
| | binding site | 0=not part of TF binding site |
| b₁₃ | Non-coding RNA (nc-RNA) | 1=part of nc-RNA ; |
| | | 0=not part of nc-RNA |
| b₁₄ | MicroRNA (mi-RNA) | 1=part of mi-RNA ; |
| | | 0=not part of mi-RNA |
| b₁₅ | Disease marker | 1=part of a disease marker ; |
| | | 0=not part of disease |
| | | marker |

To address this issue, the values of the annotation bits are suitably set to zero for the purposes of alignment processing. This can be done efficiently using a bitwise "AND" mask in which annotation bit positions are set to zero and base bit positions are set to one. For the example of Table 1, a suitable bitwise mask would be *M_{base}*=0000000000000111_{bin} (0007ₕₑₓ). Applying this mask to a character *C* of the bioinformatics character set **20** (suitably written as *C*&*M_{base}* where the ampersand indicates the bitwise "and" operation) has the effect of zeroing out all annotation bits while passing the base bits of character *C* through unaltered. Binary masking is a low-level digital processing operation and accordingly is typically quite efficient. For a base sequence represented by a string *S*=[*C₁ C₂ C₃* ... *C_{K}*] of *K* characters of the bioinformatics character set **20**, each character would need to be individually masked, for example using a loop of *K* iterations to iteratively apply mask *M_{base}* to each character *C₁,...,C_{K}* in turn. For notational convenience, this string masking opeation is represented herein by pseudocode of the form *S*&*M_{base}* where *S* is the base sequence string and *M_{base}* is the binary mask for a single character of the string *S*. Thus, to compare the base sequence fragments S₁ and S₂ for the purpose of fragment alignment (that is, comparison respective to the bases without consideration of the annotation bits), the comparison is performed between *S₁*&*M_{base}* and *S₂*&*M_{base}.*

When the sequence alignment module **22** aligns sequence fragments with reference to a reference sequence **24**, it can also identify genomic regions of significance, such as exons, introns, promoter regions, coding sequence (CDS) regions, and so forth. This is achievable if the reference sequence **24** has these regions labeled or otherwise denoted. Where the sequence alignment module **22** identifies such functional regions, it suitably sets the corresponding annotation bits to indicate these regions. Thus, for example (and again considering the illustrative bioinformatics character set of Table 1), if a given base is identified as part of an intron, then the bit b₁₀ is set to one. If a given base is identified as an exon, then bit b₉ is set to one.

Note that the sequence alignment module **22** can only update annotation bits encoding genomic regions that are identified by the alignment process. On the other hand, the sequence alignment module **22** cannot update annotation bits encoding variational information that is not determined by the alignment. For example, the sequence alignment module **22** cannot update bit b₁₅ encoding whether a base is part of a disease marker.

In some instances, the sequence alignment module **22** may fail to splice some sequence fragments into the aligned sequence. These remaining unaligned sequence fragments may be residual DNA products from host cells used in the cloning process, or may reflect errors in the sequencing processing, or may be due to other factors. These remaining unaligned sequence fragments are suitably stored as a data structure **26**, with the unaligned fragments also being represented using the bioinformatics character set **20**.

The aligned sequence which is the desired product of the sequence alignment processing is suitably stored in a sequence alignment/map (SAM) file or equivalent binary BAM file **30**. However, in the SAM file the aligned sequence is suitably represented using the bioinformatics character set **20**. Advantageously, this means that annotation information such as the base quality values (annotation bits b₆ b₅ b₄ b₃ in the example of Table 1) and functional region information (annotation bits b₁₄ b₁₃ b₁₂ b₁₁ b₁₀ b₉ b₈ b₇ in the example of Table 1) are stored directly with the bases in the genomic sequence itself within the SAM or BAM file **30**.

The aligned sequence is processed by a variational analyses module **32** which performs one or more variation analyses. These analyses are typically performed using pattern matching operations in which the aligned sequence is compared with marker patterns obtained from a signatures database **34**. The marker patterns are also preferably stored using the bioinformatics character set **20**. Bitwise masking can be used to selectively exclude or retain annotations based on their relevance to the variational analysis.

For example, consider the example of a variation that is probative when it occurs in an exon, but not elsewhere. In this case, whether the base is part of an exon is relevant, but the other annotations (e.g., base quality score) are not relevant. Whether the base is part of an exon is indicated by annotation bit b₉ in embodiment of the bioinformatics character set **20** of Table 1. The comparison is therefore suitably performed on the sequence string *S*&*M* where the bitwise mask *M*=0000001000000111_{bin} (0207ₕₑₓ). This mask retains the exon annotation bit b₉ and the base bits b₂ b₁ bo, and zeros out the remaining bits of the character. The bases of the variation signature in the database **34** have the form 0000001000000xxx_{bin} (020Xₕₑₓ) where x can be either 0 or 1 and X denotes any of 000, 001, 010, 011, or 100 (the remaining possibilities do not encode anything in the formalism of the embodiment of Table 1). Therefore, a comparison between *S&M* and the signature provides the desired pattern matching.

Advantageously, this approach implements the variational analysis using bitwise operations and character/string functions, and indeed can utilize an existing character/string functions library **36**, such as a standard character/string library provided with C++, Perl, or another programming language, or with a scripting language or so forth. Standard character or string functions are typically designed to operate on a standard character set such as ASCII or Unicode; however, if the bioinformatics character set **20** "comports" with the standard character set (e.g., ASCII or Unicode) then the character/string functions will also operate with the bioinformatics character set **20**. In this context, the bioinformatics character set **20** "comports" with ASCII or Unicode if the bioinformatics character set **20** employs the same character size (e.g., one byte for ASCII or two bytes for Unicode) and avoids any "special" characters that may have a particularly distinguished significance in ASCII or Unicode on the particular digital processing platform being utilized. For example, the null character may be utilized as an ASCII string terminator on some platforms, in which case no character of an embodiment of the bioinformatics character set **20** employing single-byte characters should have the same numerical code as the ASCII null character. Additionally, the bioinformatics character set **20** "comporting" with ASCII or Unicode in this context means that strings written in the bioinformatics character set **20** are formatted in the same way as ASCII or Unicode strings on the particular digital processing platform being utilized so as to be well-formed inputs to standard string functions. For example, on some platforms a Unicode string may include a two byte header indicating whether the characters are big-endian (typically header FEₕₑₓ, FFₕₑₓ) or little-endian (typically header FFₕₑₓ, FEₕₑₓ). In such cases, the appropriate two-byte header should be prefixed to the character string represented in the bioinformatics character set **20** prior to inputting it to a standard string function designed for Unicode.

When the variational analyses module **32** identifies a variation of interest, it suitably sets the corresponding annotation bits to indicate the variation. Thus, for example (and again considering the illustrative bioinformatics character set of Table 1), if a disease marker is identified in the genomic sequence, then the bit b₁₅ of each base matching the disease marker is set to one and this update is made in the SAM (or BAM) file **30**. As the sequence alignment module **22** already updated annotation bits identified based on the alignment processing and incuded those annotations in the SAM (or BAM) file **30**, it follows that upon completion of the variational processing the SAM (or BAM) file **30** will include all relevant functional region identification, information pertaining to variations, and base quality values in a single compact representation.

When the sequence processing (e.g., alignment and variational analyses) is completed, the results may be displayed in a human-perceptible format (e.g., displayed on a display device, printed via a printer or other marking engine, or so forth). Conventionally, such display includes listing the sequence using conventional symbols (e.g., combinations of the letters "A", "C", "G", and "T" for DNA base sequences) and providing reports summarizing variational analysis results. Instead of employing letters, in some systems other symbols are used for the bsaes. For example, in one convention adenine is represented as the symbol "×", cytosine is represented as the symbol "□",guanine is represented as the symbol "+", and thymine is represented as the symbol " ". Generating the sequence listing is conventionally straightforward because the FastA or FastQ sequence string employs a subset of ASCII. That is, the letter "A" is represented by 41ₕₑₓ in both ASCII and FastA (or Fast Q); the letter "C" is represented by 43ₕₑₓ in both ASCII and FastA (or Fast Q); the letter "G" is represented by 47ₕₑₓ in both ASCII and FastA (or Fast Q); and the letter "T" is represented by 54ₕₑₓ in both ASCII and FastA (or Fast Q). Thus, the FastA (or FastQ) base string is an ASCII string, and can be printed using any font mapping to ASCII.

The sequence alignment/analysis module **14**, on the other hand, uses the bioinformatics character set **20** which is different from ASCII (or Unicode), although it preferably comports with ASCII (or Unicode, for two-byte character set embodiments). As a consequence, although the genomic or proteomic data represented using the bioinformatics character set **20** could formally print using a standard font that maps to ASCII (or Unicode in two-byte character embodiments), the displayed data would appear to be nonsensical. Accordingly, the sequence alignment/analysis module **14** includes a bioinformatics font **40** mapped to the bioinformatics character set **20**. The font **40** provides a font character that is displayed for each character of the bioinformatics character set **20**. A sequence analysis results display module **42** suitably displays genomic or proteomic sequences represented in the bioinformatics character set **20** using the bioinformatics font **40**. The font characters of the bioinformatics font **40** preferably include (1) a letter to represent the base or peptide (or optionally a three-letter sequence to represent a peptide) and (2) additional characteristics, such as diacritical marks, font style aspects such as boldface and/or italic font style, or so forth to represent the annotated data associated with the base or peptide in the representing character of the bioinformatics character set **20**. Instread of using letters, the base or peptide repesentation (1) may employ another type of symbol, such as: adenine = "×", cytosine="□",guanine="+", and thymine=" ". Advantageously, the sequence analysis results display module **42** can utilize conventional text display routines provided by the platform to display ASCII or Unicode text. These text display routines are adapted to display the genetic sequence simply by invoking the display routine to display (or print) using the bioinformatics font **40**.

The sequence alignment/analysis module **14** can be embodied as a digital processing device, such as an illustrative computer **50**, that includes a digital processor (not shown) programmed to execute software implementing the various modules **16**, **22**, **32** and including memory storing the bioinformatics font **40**. Rather than the illustrative computer **50**, another digital processing device can be used, such as a dedicated DNA sequencing apparatus that includes a digital processor, or a network server system, or a graphical processing unit (GPU) such as a gaming machine reprogrammed to implement the sequence alignment/analysis module **14**, or so forth. The sequence alignment/analysis module **14** optionally includes or has access to a display device (such as an illustrative display **52** of the computer **50**) for displaying information such as genomic or proteomic sequences represented using the bioinformatics font **40**.

The sequence alignment/analysis module **14** can also be implemented as a non-transitory storage medium storing the bioinformatics font **40** and software that when executed by a digital processor (such as the processor of the computer **50**) implements the various modules **16**, **22**, **32**. Such a non-transitory storage medium may, by way of illustrative example, include one or more of the following: a hard drive or other magnetic storage medium; an optical disk or other optical storage medium; a read-only memory (ROM), random access memory (RAM), flash memory, or other electrostatic memory or combination of electrostatic memories; or so forth.

Diagrammatic FIGURE 1 illustrating the sequence alignment/analysis module **14** diagrammatically denotes the bioinformatics character set **20**. However, it is to be appreciated that in some embodiments the bioinformatics character set **20** is embodied implicitly by the processing performed by the various modules **16**, **22**, **32** that create and manipulate genomic or proteomic sequences formatted using the bioinformatics character set **20**. On the other hand, the bioinformatics font **40** is stored as a set of bitmaps or other font character representations. In some embodiments it is contemplated to construct the bitmap for a given font character "as needed" by combining or modifying stored constituent feature bitmaps, for example by adding one or more diacritical marks representing annotated data to a bitmap depicting a letter representing the base or peptide.

Having described the illustrative sequence alignment/analysis module **14** with reference to FIGURE 1 and the embodiment of the bioinformatics character set **20** given in Table 1, some additional embodiments of the bioinformatics character set **20** and some illustrative bioinformatic font characters are next described.

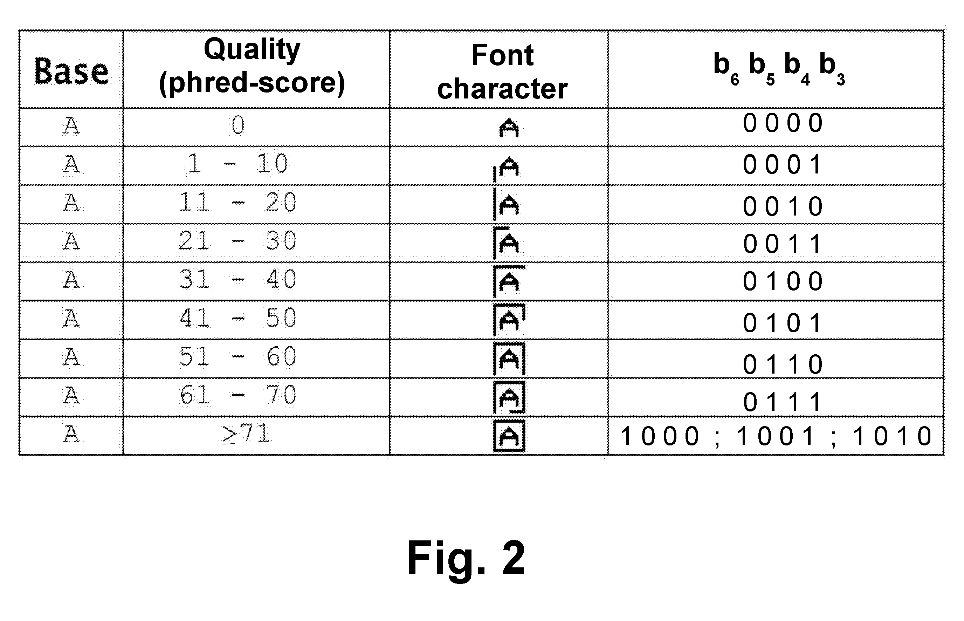

With reference to FIGURE 2 and Table 1, some font characters suitable for displaying characters of the bioinformatics character set **20** including both base and base quality value information are shown. In this approach, diacritical marks indicating base quality values are determined from the base quality annotation bits b₆ b₅ b₄ b₃ and comprise a set of one or more joined (as illustrated in FIGURE 2) or separated line segments whose total length is indicative of the base quality value. Note that in the embodiment of FIGURE 2 the diacritical annotation values 1000_{bin}, 1001_{bin}, and 1010_{bin} are all mapped to the same font character. Thus, that font character indicates a phred score of greater than 70. The diacritical marks of FIGURE 2 are shown for the base adenine indicated by the letter "A". More generally, in a suitable embodiment the base adenine is indicated by the letter "A" or "a"; the base cytosine is indicated by the letter "C" or "c"; the base guanine is indicated by the letter "G" or "g"; the base thymine is indicated by the letter "T" or "t"; and the base uracil is indicated by the letter "U" or "u". The diacritical marks (i.e., lines) of FIGURE 2 are readily applied to any of these letters. Advantageously, a reviewer reading bases represented by the font characters shown in FIGURE 2 can readily assess the quality value of each base in the sequence.

It should be noted that, as used herein, the phrase "the letter 'A' or 'a"' indicates a symbol recognizable as the letter "A" or "a" as would be understood by an ordinary person. The letter "A" or "a" may be variously represented, for example using an Arial type face, or a Times New Roman type face, or a Courier type face, or a handwritten type face, or so forth. Analogous comments apply for other letters indicative of bases or peptides in the bioinformatics font **40**.

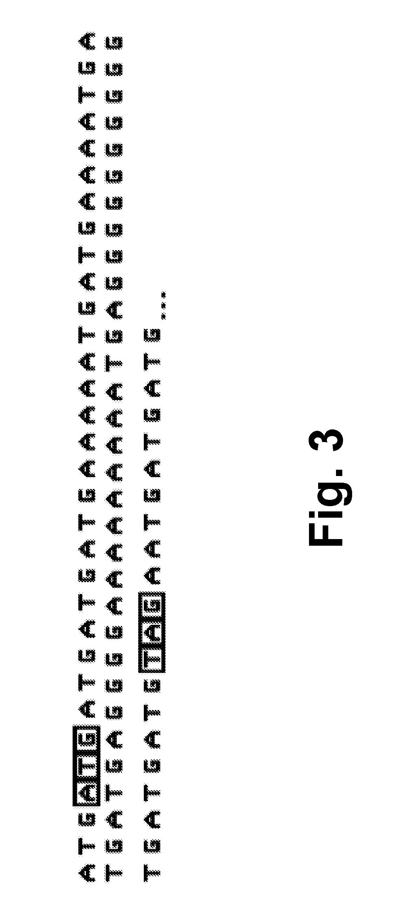

With reference to FIGURE 3 and Table 1, a font is shown for representing coding sequence (CDS) start and stop codons. In the character set embodiment of Table 1, bases are annotated as CDS start or CDS stop codons via annotation bits b₇ and b₈, respectively. In the font embodiment of FIGURE 3, characters having either b₇ or b₈ equal to one are mapped to font characters that include a box around the letter representing the base; whereas, characters having both b₇ and b₈ equal to zero are mapped to font characters that do not include such a box. As seen in FIGURE 3, the result of this font mapping is that the start and stop codons are readily recognized by the diacritical box marks. In the embodiment of FIGURE 3, the same diacritical box mark is used for both CDS start and CDS stop; alternatively, different diacritical marks can be used for CDS start and CDS stop, which could assist the reviewer in discerning the beginning versus end of a CDS.

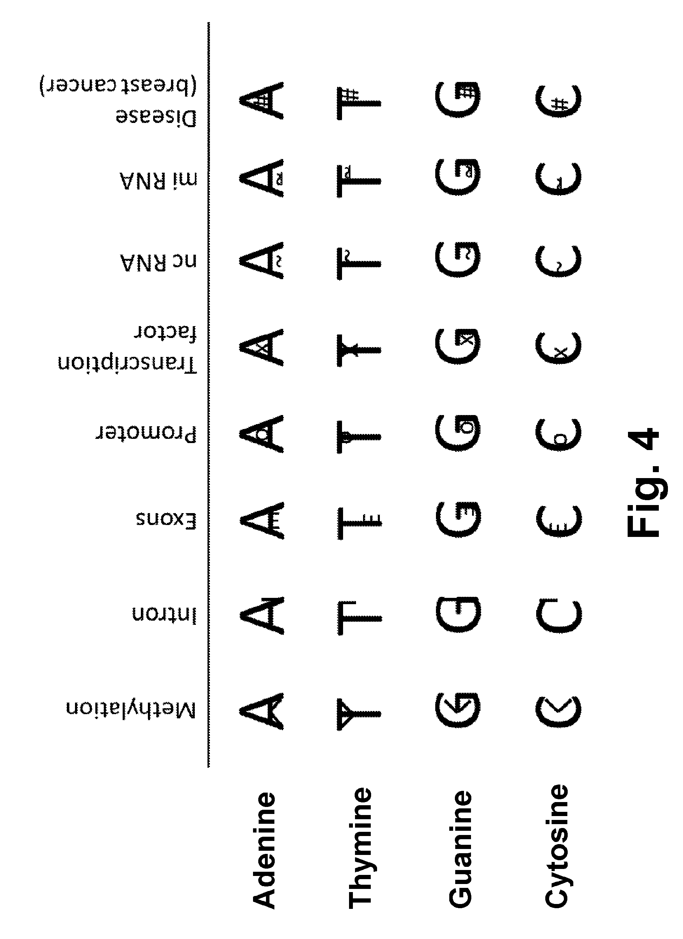

With reference to FIGURE 4, some other suitable diacritical marks for indicating various genomic regions or variations are shown. In the examples of FIGURE 4, characters of the bioinformatics character set **20** that include an annotation indicating methylation are mapped to font characters of the bioinformatics font **40** that include diacritical marks comprising an angle symbol (i.e., like a "V" but optionally with the point of the "V" oriented other than downward). In another example shown in FIGURE 4, characters of the bioinformatics character set **20** that include an annotation indicating an intron (e.g., b₁₀ in the example of Table 1 being set to one) are mapped to font characters of the bioinformatics font **40** that include diacritical marks comprising the letter "I" or "i". In another example shown in FIGURE 4, characters of the bioinformatics character set **20** that include an annotation indicating an exon (e.g., b₉ in the example of Table 1 being set to one) are mapped to font characters of the bioinformatics font **40** that include diacritical marks comprising the letter "E" or "e". In another example shown in FIGURE 4, characters of the bioinformatics character set **20** that include an annotation indicating a promoter (e.g., b₁₁ in the example of Table 1 being set to one) are mapped to font characters of the bioinformatics font **40** that include diacritical marks comprising the letter "P" or "p". In another example shown in FIGURE 4, characters of the bioinformatics character set **20** that include an annotation indicating a transcription factor (TF) binding site (e.g., b₁₂ in the example of Table 1 being set to one) are mapped to font characters of the bioinformatics font **40** that include diacritical marks comprising the letter "X" or "x". In another example shown in FIGURE 4, characters of the bioinformatics character set **20** that include an annotation indicating a region of non-coding RNA (e.g., b₁₃ in the example of Table 1 being set to one) are mapped to font characters of the bioinformatics font **40** that include diacritical marks comprising "~". (In other contemplated embodiments, diacritical marks comprising the letters "NC" or "nc" or "Nc" are contemplated for indicating nc-RNA). In another example shown in FIGURE 4, characters of the bioinformatics character set **20** that include an annotation indicating a region of microRNA (e.g., b₁₄ in the example of Table 1 being set to one) are mapped to font characters of the bioinformatics font **40** that include diacritical marks comprising "≃". (In other contemplated embodiments, diacritical marks comprising the letters "MI" or "mi" or "Mi" are contemplated for indicating mi-RNA). In another example shown in FIGURE 4, characters of the bioinformatics character set **20** that include an annotation indicating the base is part of a disease marker (e.g., b₁₅ in the example of Table 1 being set to one) are mapped to font characters of the bioinformatics font **40** that include diacritical marks comprising "#" or some other diacritical mark designated to indicate a disease marker. Additionally or alternatively, the disease marker annotation can be indicated by depicting the base letter (e.g., "A" in the case of adenine) using a designated disease-marking font style such as an italic font style, a boldface font style, or an italic boldface font style.

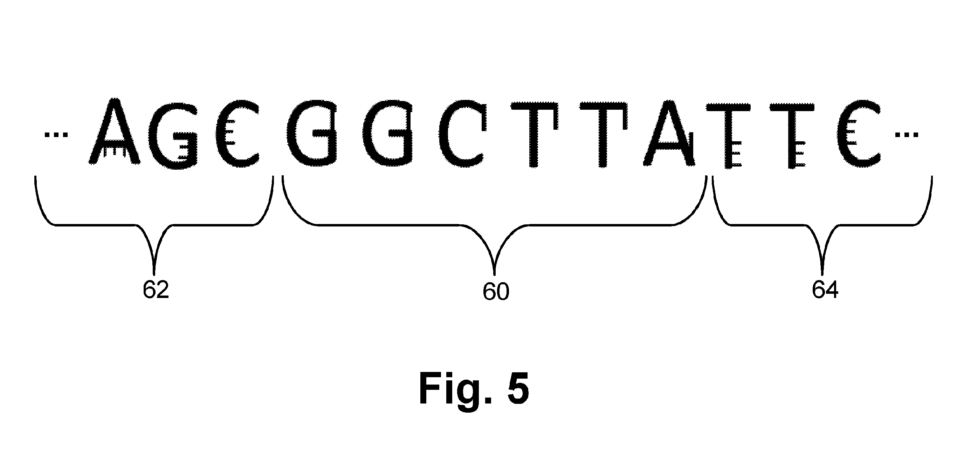

FIGURE 5 shows an example of the efficacy of such font characters in delineating sequence regions. FIGURE 5 depicts a portion of a DNA sequence represented using fonts of FIGURE 4. An intron region **60** bounded by two exon regions **62**, **64** is readily discernable based on the diacritical marks "I" and "E" denoting bases belonging to introns and exons, respectively.

The diacritical marks of FIGURES 4 and 5 are merely illustrative examples, and other diacritical marks as well as other font characteristics such as font style, font size, or so forth may also be employed in the bioinformatics font **40** to denote various annotations. For example, in another contemplated approach the base quality value is depicted by the size of the letter depicting the base, with larger letters indicating higher base quality values. The expectation in this approach is that the reviewer will likely naturally associate a smaller letter with lower base quality, i.e. higher uncertainty. As some other examples: strand information (5', 3', +, -, and so forth) can be stored as an annotated datum value in characters of the bioinformatics character set **20** and can be suitably represented by font character diacritical marks; variants such as insertions and/or deletions (generally, "indels") can be stored as an annotated datum value in characters of the bioinformatics character set **20** and can be suitably represented by font character diacritical marks such as vertical, horizontal, or slanted strikethrough marks (for deletions) or carat marks (that is, "^") (for insertions, following the textual editor's markup convention), or by using hollowed out or filled stroked font characters; and so forth.

Various diacritical marks indicative of different types of annotated data can be combined in the bioinformatics font **40** in order to simultaneously convey different types of information when displaying a base or peptide sequence represented by the bioinformatics character set **20** using the bioinformatics font **40**. For example, (again referring to the embodiment of the bioinformatics character set **20** of Table 1) the character 0000001000110000_{bin} (0230ₕₑₓ) denotes an adenine base having base quality value in the range 51-60 which is part of an exon. On the other hand, the character 1000001000110000_{bin} (8230ₕₑₓ) denotes an adenine base having base quality value in the range 51-60 which is part of an exon which is also part of a disease marker. The mapped font character for the latter character suitably differs from the mapped font character for the former character only in the addition of a diacritical mark indicating the attribute of being part of a disease marker. Additionally, the base quality value may be depicted using a suitable diacritical mark such as those shown in FIGURE 2. If several different types of base or peptide attributes or characteristics are similarly encoded, then the number of font characters in the bioinformatics font **40** can be quite large. For one byte characters, there may be as many as 256 different font characters, while for two byte characters there may be as many as 65,536 font characters.

Referring back to the example of Table 1, the various annotations are (with the exception of base quality value) represented by a single bit for each annotation. Thus, for example, a value of one for bit b₁₀ indicates the base is part of an intron, while a value of zero for bit b₁₀ indicates the base is not part of an intron. However, this representational approach has a potential drawback, in that if bit b₁₀ has a value of zero it may be ambiguous whether this is (1) an affirmative representation that the base is not part of an intron, or (2) an indication that it has not (yet) been determined whether or not the base is part of an intron. This is because the format conversion module **16** assigns a default value of zero for the annotation bits (other than those indicating base quality value).

With reference to Table 2, another illustrative embodiment of the bioinformatic character set **20** is shown which overcomes this ambiguity by providing a distinct value of an annotation for an unknown value. In this embodiment the intron annotation is represented by two bits b₇ b₆. A value of 01_{bin} indicates the base is not part of an intron; a value of 10_{bin} indicates the base is part of an intron; and a value of 11_{bin} indicates that it is not (yet) known whether the base is or is not part of an intron.

The example of Table 2 illustrates some other contemplated features of certain embodiments of the bioinformatics character set **20**. The example of Table 2 employs a single-byte character (whereas the example of Table 1 employs a two-byte character). The example of Table 2 also uses four bits b₃ b₂ b₁ b₀ to represent the base, which enables more detailed representation of ambiguous bases. The base representation also uses an encoding scheme that associates specific bits with specific bases. Thus, bit b₃ is associated with thymine; bit b₂ is associated with guanine; bit b₁ is associated with cytosine; and bit b₀ is associated with adenine. With this encoding scheme, an unambiguous base is represented by a single bit of the four bits b₃ b₂ b₁ b₀ having value one. Ambiguity between two possible bases is represented by two bits of the four bits b₃ b₂ b₁ b₀ having value one, thus identifying the two possibilities for the base. The endpoint case of complete ambiguity is represented by all four bits b₃ b₂ b₁ b₀ having value one, indicating the base could be any of adenine, cytosine, guanine, or thymine. This encoding scheme also enables rapid matching of ambiguous bases to a pattern. For example, by using a bitwise mask 04ₕₑₓ, a character *C* can be compared with the base guanine represented by 04ₕₑₓ by the comparison CMP[*C*&04ₕₑₓ , 04ₕₑₓ] (where CMP[...] is pseudocode for a comparison operation). This comparison will yield a match if the character *C* unambiguously encodes for guanine, and will also yield a match if the character *C* is ambiguous but encodes guanine as a possible value - in both cases *C*&04ₕₑₓ = 04ₕₑₓ.

**Table 2**

| **Bioinformatics character** | **Quantity stored** | **Allowable values** |
|---|---|---|
| **b₇ b₆ b₅ b₄ b₃ b₂ b₁ b₀** | | |
| Bits 0-3 | Base | 0001=adenine; 0010=cytosine; |
| | | 0100=guanine; 1000=thymine; |
| | | 0011=adenine or cytosine; |
| | | 0101=adenine or guanine; |
| | | 1001=adenine or thymine; |
| | | 0110=cytosine or guanine; |
| | | 1010=cytosine or thymine; |
| | | 1100=guanine or thymine; |
| | | 0111=adenine or cytosine or guanine; |
| | | 1011=adenine or cytosine or thymine; |
| | | 1101=adenine or guanine or thymine; |
| | | 1110=cytosine or guanine or thymine; |
| | | 1111=unknown |
| Bits 4-5 | Coding | 10=CDS start ; |
| | sequence | 01=CDS end ; |
| | (CDS) | 00=neither CDS start nor CDS end |
| | | 11=unknown |
| Bit 6-7 | Intron | 00=not used |
| | | 01=not part of an intron |
| | | 10=part of an intron |
| | | 11=unknown |

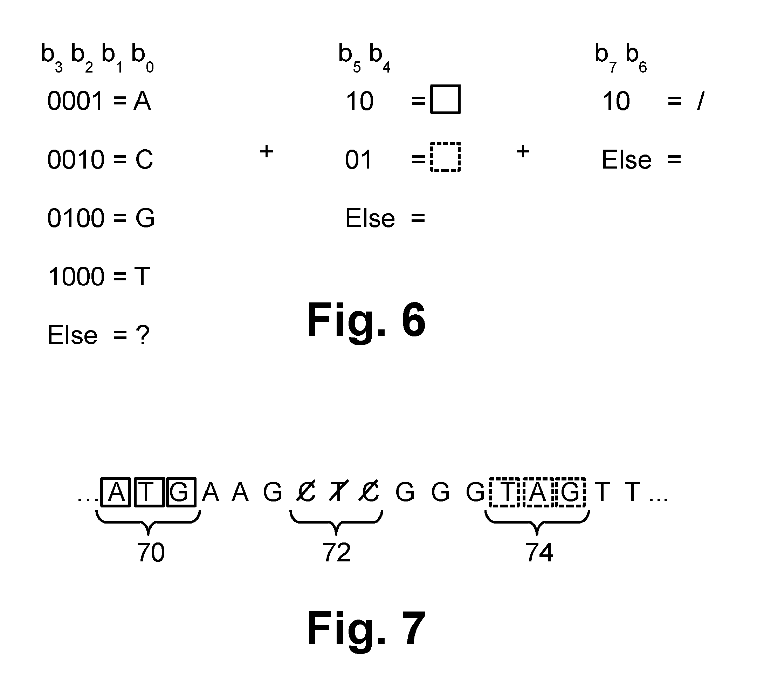

With reference to FIGURES 6 and 7 and continuing reference to the example of Table 2, in some embodiments the font characters of the bioinformatics font **40** are not stored directly. Instead, bitmaps of the base or peptide letter (or three-letter) representations are stored, along with bitmaps representing the various diacritical marks for different types of annotations. The font character for depicting a particular character of the bioinformatics character set **20** can then be constructed by combining these constituent bitmaps using a logical "OR" operation. In FIGURE 6 the leftmost term shows the bitmaps encoding the four bases (with any ambiguous base represented by the symbol "?"). The middle term shows bitmaps representing the diacritical marks for CDS start (represented by a solid box) and CDS end (represented by a dotted box). The rightmost term shows bitmaps representing the diacritical mark for an intron (represented as a slash crossing through the letter representing the base). The plus (+) symbols in FIGURE 6 indicate logical OR of the bitmaps (having a logic table: black dot+black dot=black dot; black dot+white dot=black dot; white dot+black dot=black dot; white dot+white dot=white dot). FIGURE 7 shows a partial base sequence depicted using embodiment of the bioinformatics font **40** shown in FIGURE 6, including a CDS start region **70**, an intron region **72**, and a CDS end region **74**. The diacritical mark "/" indicating a base belonging to an intron intuitively informs the reviewer that the intron is not included in the translated protein.

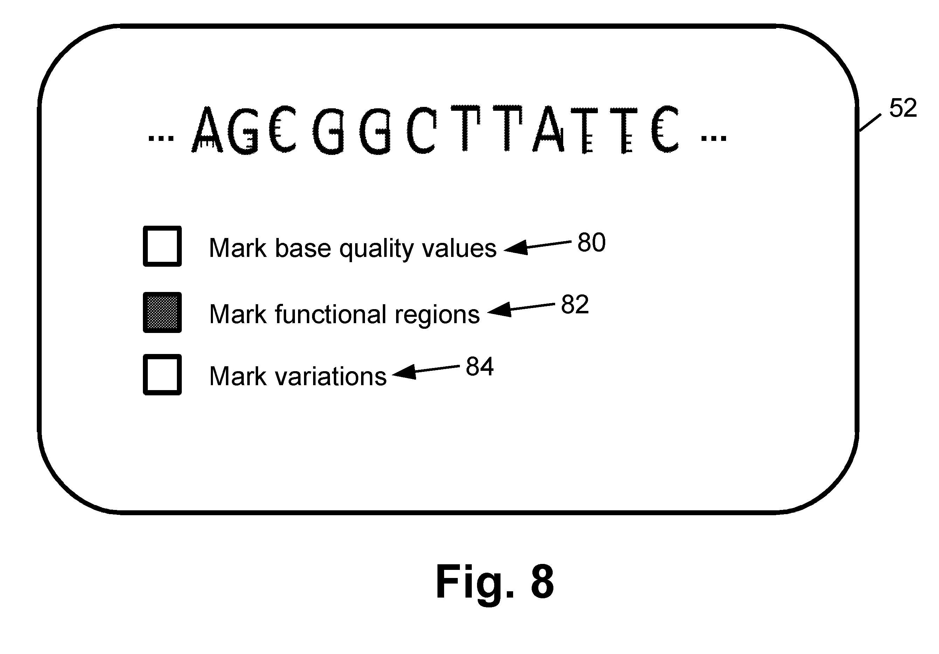

With reference to FIGURE 8, yet another advantage of the disclosed sequence alignment/analysis module **14** is that it is computationally convenient to enable the user to select which annotation(s) to view when depicting a genomic or proteomic sequence. Such selective depiction of annotation types can be useful to enable the reviewer to focus on a particular aspect of the sequence. The example of FIGURE 8 employs the embodiment of the bioinformatics character set **20** of Table 1. FIGURE 8 shows a portion of a genomic sequence displayed on the display device **52** (e.g., the display of the computer **50** of FIGURE 1). The display also shows user dialog selection options, including a check box **80** via which the reviewer selects whether to display base quality values (e.g., using diacritical marks such as those shown in FIGURE 2), a check box **82** via which the reviewer selects whether to display functional regions (e.g., using diacritical marks for showing genomic regions such as those shown in FIGURE 4), and a check box **84** via which the reviewer selects whether to display variations (e.g., using diacritical marks to highlight disease markers such as those shown in FIGURE 4). The user suitably checks or unchecks the various check boxes **80**, **82**, **84** using a pointer input device (e.g., a mouse, track ball, trackpad, or so forth), a keyboard (e.g., using the tab key to cycle through the options and pressing enter to toggle a selected check box), or so forth. In FIGURE 8, the user has selected to show only the functional regions via check box **82**.

Display options such as those shown in FIGURE 8 are readily implemented using the disclosed bioinformatics character set **20** and bioinformatics font **40** by using bitwise masking to remove annotations that are not to be displayed prior to inputting the character string to the text display routine (e.g., provided by the platform to display ASCII or Unicode text). For example, considering the example of Table 1, a suitable bitwise mask for displaying only the functional regions but not base quality values or disease markers is *M_{display}*=0111111110000111_{bin} (7F87ₕₑₓ), which sets the base quality value (bits b₆ b₅ b₄ b₃) to 0000_{bin} and sets the disease marker annotation (bit b₁₅) to zero. This approach assumes that the bioinformatics font **40** displays the lowest base quality value (i.e., 0000_{bin}) with no diacritical mark, as is the case for the illustrative approach of FIGURE 2. Note that this masking is applied prior to input to the text display routine, but the mask output is not used to update the stored sequence **30**. Thus, in the data file **30** the base quality values and disease marker annotations remain unaltered, and therefore the reviewer can choose to update the display options at any time. For example, the reviewer might elect to turn off the functional region markings and turn on the variation markings by unchecking box **82** and checking box **84**, respectively. The updated display would be readily implemented by updating the mask to *M_{display}*=10000000000000111_{bin} (8007ₕₑₓ), which sets the base quality value (bits b₆ b₅ b₄ b₃) to 0000_{bin} and sets the functional region annotation (bits b₁₄ b₁₃ b₁₂ b₁₁ b₁₀ b₉ b₈ b₇) to zero.

The illustrative embodiments have employed genomic data, and more particularly DNA data utilizing the four bases adenine, cytosine, guanine, and thymine. However, the disclosed approaches employing the bioinformatics character set **20** and bioinformatics font **40** are readily employed for other genomic data such as RNA sequences by replacing thymine with uracil.

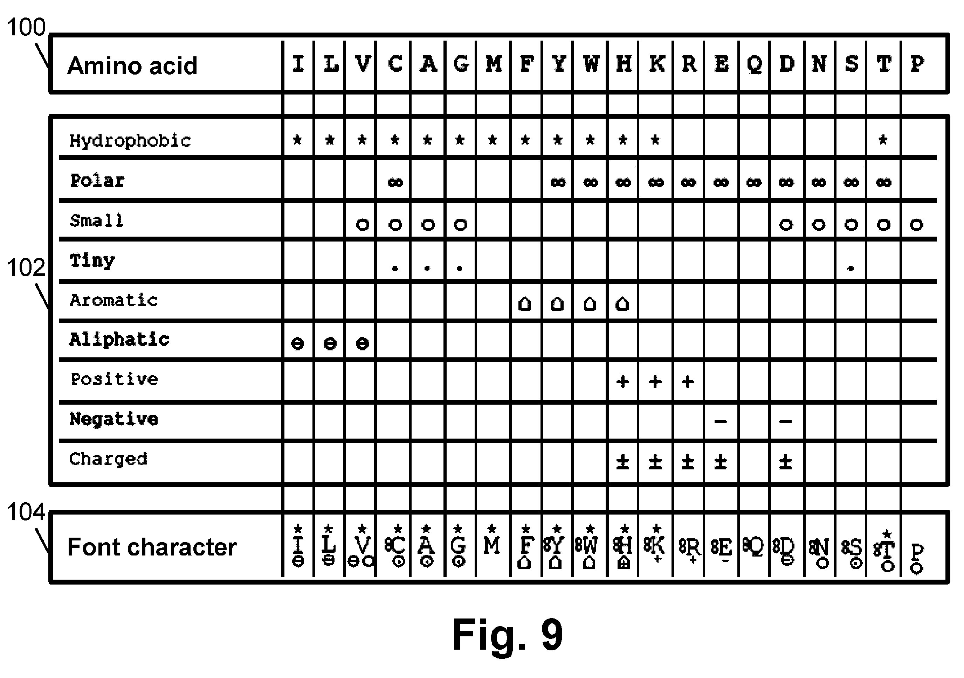

With reference to FIGURE 9, the disclosed approaches employing the bioinformatics character set **20** and bioinformatics font **40** are also readily employed for proteomic data by employing a suitable number of bits to represent the amino acid (or peptide). Proteins are constructed from a set of twenty amino acids, which can be represented using five bits having 32 possible values. Five bits is thus also sufficient to represent any additional peptides that may be of interest, such as selenocysteine (conventionally represented by the letter "U" or "u" or by the three-letter code "sec") and pyrrolysine ("O" or "o" or "Pyl") which can be incorporated by overriding the CDS end codon, and/or to represent ambiguous amino acids (of which typically only a half dozen or fewer pairwise ambiguities may arise for most proteomic sequencing techniques). FIGURE 9 illustrates construction of font characters of the bioinformatics font **40** representing various illustrative peptides or amino acids that include various annotated data by combining a bitmap for the amino acid or peptide with one or more bitmaps for one or more diacritical marks using a bitwise OR operation. In FIGURE 9, the topmost section **100** specifies a suitable single-letter coding of the twenty amino acids. The middle section **102** of FIGURE 9 specifies various combinations of annotated data that may be associated with a peptide of the proteomic sequence. In the illustrative example of FIGURE 9, the attributes that may be annotated to a peptide include: hydrophobic - indicated by a superscript star (*); polar - indicated by a preceding "∞" diacritical mark; small - indicated by a subscript "o"; tiny - indicated by a subscript dot; aromatic - indicated by a subscript "house" representation ( 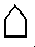 ); aliphatic - indicated by a subscript theta ( ); positive - indicated by a subscript "+"; negative - indicated by a subscript "-"; and charged (a generalization of the positive and negative characteristics). The bottom section **104** of FIGURE 9 shows the font character generated by combining the bitmap for the amino acid or peptide from upper section **100** with the bitmap or bitmaps for the diacritical mark or marks representing the annotated datum or data using a bitwise OR operation. In the embodiment of the bioinformatics font **40** shown in FIGURE 9, the diacritical marks are chosen such that combinations of annotated data can be represented simultaneously by combinations of diacritical marks provided by the bitwise OR operation applied to the bitmaps **100**, **102**. Thus, by way of illustrative example, the leftmost column of FIGURE 9 shows the amino acid isoleucine (I) having the attributes of being hydrophobic and aliphatic. The corresponding font character shown in the lower section **104** includes a superimposition of the character set representing the amino acid symbol (that is, the letter "I") with a superscript asterisk (*) representing the hyrodophobic property and a subscript theta ( ) representing the aliphatic property.

It should be noted that, in general, the bioinformatics character set **20** and associated bioinformatics font **40** will be specific to genomic data or to proteomic data. In other words, a given embodiment of the bioinformatics character set **20** and associated bioinformatics font **40** will be designed to represent either genomic data, or proteomic data, but typically not both genomic and proteomic data. (Embodiments in which a "combination" character set and associated font capable of representing both genomic and proteomic data is contemplated, but typically using a designated genomic or proteomic character set and font enables construction of more efficient bioinformatic character sets and more intuitive bioinformatic fonts).

It should further be noted that a given bioinformatic character set **20** may have two more different bioinformatic fonts **40** associated therewith. By way of illustrative analogy, in the same way that the characters of the ASCII character set can be represented by different fonts (e.g., Times New Roman font, Arial font, and so forth), different bioinformatic fonts **40** can be used to represent a single bioinformatic character set **20**. The user may then select his or her preferred representation of the genomic or proteomic sequence simply by selecting the user's preferred bioinformatic fonts **40**, in the same way that a reader may select to display or print English text using Times New Roman font, Arial font, or any other available ASCII font that the reader may prefer.

The disclosed approaches employing the bioinformatics character set **20** and bioinformatics font **40** are still further readily employed to accommodate additional or other annotation types. In the case of genomic sequences, annotations of interest include (but are not limited to): methylation, acetylation, CDS start and end, Exon start and end, Intron start and end, promoter, enhancer, TF, pseudogenes, STS, D-loop, V-loop, miRNA, piRNA, ncRNA, repeats (LINE, SINE, etc), GAP, disease specific signatures, and so forth. In the case of proteomic sequences, annotation types of interest may include variations such as a single nucleotide variant (SNV) or substitution, tertiary structure information, or so forth.

In the illustrated examples of Tables 1 and 2, only one variation annotation is illustrated (the disease marker bit b₁₅ of the example of Table 1). However, it is to be appreciated that more and/or different variation annotations may be included in the bioinformatics character set **20**. For example, different annotations may be provided for different disease markers, or annotations may be provided for variations of other types such as variations indicative of anscestral lineages or variations associated with particular phenotype traits.

This application has described one or more preferred embodiments.

Modifications and alterations may occur to others upon reading and understanding the preceding detailed description. It is intended that the application be construed as including all such modifications and alterations insofar as they come within the scope of the appended claims or the equivalents thereof.

## Claims

1. A method comprising:
encoding genomic or proteomic data as a genomic or proteomic character string comprising characters of a bioinformatics character set wherein:
(i) each base or peptide of the genomic or proteomic data is represented by a single character of the bioinformatics character set **(20)** and
(ii) each character of the bioinformatics character set encodes (I) a base or
peptide and (II) at least one annotated datum value associated with the base or peptide; and
displaying the genomic or proteomic data by displaying the genomic or proteomic character string using a bioinformatics font **(40)** mapped to the bioinformatics character set;
wherein the encoding and displaying are performed by a digital processing device (**50**).

2. The method of claim **1** wherein each character of the bioinformatics character set is represented by one of (1) a single byte consisting of eight bits and (2) two bytes consisting of sixteen bits, wherein a first sub-set of the eight or sixteen bits encodes a base or peptide and a second sub-set of the eight or sixteen bits encodes at least one annotated datum value associated with the base or peptide.

3. The method of any one of claims **1-2** wherein:
each character of the bioinformatics character set that encodes an adenine base is mapped to a font character of the bioinformatics font that includes the letter "A" or "a",
each character of the bioinformatics character set that encodes an guanine base is mapped to a font character of the bioinformatics font that includes the letter "G" or "g",
each character of the bioinformatics character set that encodes an cytosine base is mapped to a font character of the bioinformatics font that includes the letter "C" or "c",
each character of the bioinformatics character set that encodes an thymine or
uracil base is mapped to a font character of the bioinformatics font that includes the letter "T" or "t" or the letter "U" or "u"; and
at least one character of the bioinformatics character set encodes an ambiguous base using a code representing two or more candidate bases.

4. The method of claim **3** wherein:
each character of the bioinformatics character set encodes an annotated datum value indicating a quality value of the encoded base and
the bioinformatics font includes diacritical marks indicating base quality values.

5. The method of claim **1** wherein at least four characters of the bioinformatics character set are mapped to font characters of the bioinformatics font that each include one or more letters representing the base or peptide encoded by the character and one or more diacritical marks representing the encoded at least one annotated datum.

6. The method of any one of claims **1-5** further comprising:
performing at least one string function on the genomic or proteomic character string to generate an updated genomic or proteomic character string in which at least one base or
peptide is represented by a single character encoding at least one additional or modified annotated datum generated by the performed string manipulation.

7. The method of claim **6** wherein the performing includes performing a string comparison comparing the genomic or proteomic character string with a reference genomic or proteomic character string.

8. The method of any one of claims **6-7** wherein the performing includes performing a bitwise logical operation on the characters of the genomic or proteomic character string.

9. The method of any one of claims **1-8**, wherein the method encodes only genomic data and comprises:
encoding genomic data as a genomic character string comprising characters of a bioinformatics character set wherein:
(i) each base of the genomic data is represented by a single character of the bioinformatics character set **(20)** and
(ii) each character of the bioinformatics character set encodes (I) a base and
(II) at least one annotated datum value associated with the base; and
displaying the genomic data by displaying the genomic character string using a bioinformatics font **(40)** mapped to the bioinformatics character set.

10. The method of any one of claims **1-8**, wherein the method encodes only proteomic data and comprises:
encoding proteomic data as a proteomic character string comprising characters of a bioinformatics character set wherein:
(i) each peptide of the proteomic data is represented by a single character of the bioinformatics character set **(20)** and
(ii) each character of the bioinformatics character set encodes (I) a peptide and
(II) at least one annotated datum value associated with the peptide; and
displaying the proteomic data by displaying the proteomic character string using a bioinformatics font **(40)** mapped to the bioinformatics character set.

11. An apparatus comprising:
a digital processing device **(50)** configured to perform a method as set forth in any one of claims **1-10**.

12. A non-transitory storage medium readable by a digital processor and storing software for processing genomic or proteomic data represented as genomic or proteomic character strings comprising characters of a bioinformatics character set **(20)** wherein each base or peptide of the genomic or proteomic data is represented by a single character of the bioinformatics character set and the characters of the bioinformatics character set encode bases or peptides and additional data associated with the bases or peptides.

13. The storage medium as set forth in claim **12**, wherein the software processes the genomic or proteomic data using string processing operations.

14. The storage medium as set forth in any one of claims **12-13**, wherein the software processes the genomic or proteomic data using bitwise masking operations to zero selected binary bits of characters representing bases or peptides.

15. The storage medium as set forth in any one of claims **12-14**, wherein the storage medium further stores a bioinformatics font **(40)** mapped to the bioinformatics character set, and the software performs display operations in which genomic or proteomic data are displayed using the bioinformatics font.
